# EUROPÄISCHE PATENTSCHRIFT

(11) **EP 3 017 097 B1**
(45) Veröffentlichungstag und Bekanntmachung des Hinweises auf die Patenterteilung: **08.01.2020**
(21) Anmeldenummer: 14724641.7
(22) Anmeldetag: 07.05.2014
(51) Int. Cl.: D01G 27/00, B65H 18/20, B65H 18/26

(54) **WICKELMASCHINE ZUM AUFWICKELN VON WATTENBÄNDERN ZU WATTEWICKELN**
LAP WINDER FOR WINDING STRIPS OF COTTON WOOL TO LAP ROLLS
ENROULEUSE DESTINÉE À ENROULER DES BANDES DE NAPPE POUR OBTENIR DES ROULEAUX DE NAPPE

(30) Priorität: 04.07.2013 DE 102013107031; 07.08.2013 DE 102013108507
(43) Veröffentlichungstag der Anmeldung: 11.05.2016
(73) Patentinhaber: Trützschler GmbH & Co. KG, 41199 Mönchengladbach (DE)
(72) Erfinder: SCHMITZ, Thomas, 41238 Mönchengladbach (DE)
(86) Internationale Anmeldenummer: PCT/EP2014/001208
(87) Internationale Veröffentlichungsnummer: WO 2015/000537

(56) Entgegenhaltungen:
- EP-A2- 2 080 722
- CN-Y- 2 677 393
- DE-A1- 19 539 365
- DE-A1- 19 634 748
- GB-A- 406 483
- US-A- 3 000 584
- US-A- 3 858 820

## Beschreibung

Die Erfindung betrifft eine Wickelmaschine zum Aufwickeln von Wattebändern zu Wattewickeln, mit Wickelwalzen zum Tragen jeweils eines Wattewickels, mit den Wickelwalzen vorgeschalteten Druckwalzen zum Kalandrieren eines zugeführten Wattenbandes, wobei den Druckwalzen mindestens ein Streckwerk mit Streckwerkswalzenpaaren vorgelagert ist.

Die heute übliche Speisung von Flachkämmmaschinen erfolgt durch Wattewickel, die zuvor in Wickelmaschinen aus einzelnen Bändern erzeugt wurden. Dazu erhält die Wickelmaschine die Vorlage in Bandform von mindestens einer Strecke, wobei die Vorlage in runden oder rechteckigen Kannen zwischengespeichert wird. Die Wickelmaschine besteht üblicherweise aus einer Wickeleinheit mit mindestens zwei Wickelwalzen, auf denen mittels einer Hülse der Wattewickel gebildet wird. Der Wickeleinheit vorgelagert sind in der Regel mindestens ein Paar Druckwalzen, die die Bänder doublieren und/oder verstrecken. Vor den Druckwalzen ist ein Einlaufbereich angeordnet, indem eine weitere Verdichtungseinheit oder ein Glätter angeordnet sein kann.

Die DE 19634748 beschreibt eine Wickelmaschine, bei denen die Wickelwalzen in einem festen Drehzahlverhältnis über einen Antriebsmotor angetrieben werden. Um das Drehzahlverhältnis der Wickelwalzen zu ändern, ist der Austausch von Zahnrädern erforderlich, wozu die Wickelmaschine gestoppt werden muss.

In der DE 102008000179 A1 ist ein Wickler für Warenbahnen beschrieben, bei denen jede Wickelwalze einen eigenen Antriebsmotor aufweist. Zur Reduzierung von Schwingungen werden beide Wickelwalzen mit gleicher Drehzahl betrieben und je nach Größe der Schwingungen in der Drehzahl reduziert.

Die CN 2677393 beschreibt eine Wickelmaschine, bei der über einen einstellbaren Pneumatikzylinder der Wickeldruck über die Wickellänge steigt. Um einen Verzug zwischen den Wickelwalzen einzustellen, kann mit einer Änderung des Getriebes das Drehzahlverhältnis der Wickelwalzen eingestellt werden.

In der US 3,000,584 wird eine Wickelmaschine für Papier beschrieben, bei der eine erste Wickelwalze statisch mit einer bestimmten Drehzahl über einen Elektromotor angetrieben wird. Vom Elektromotor wird indirekt Antriebsleistung bzw. eine Drehzahl abgezweigt, die über ein variables Getriebe die zweite Wickelwalze antreibt. Die Drehzahldifferenz zwischen den Wickelwalzen wird über den zunehmenden Durchmesser des Wickels, der sich damit langsamer auf den Wickelwalzen dreht, abgegriffen.

Die Qualität des erzeugten Wickels ist mitentscheidend für die Produktivität der nachfolgenden Kämmmaschine. Eine hohe Gleichmäßigkeit, geringe Haarigkeit und ein gutes Abrollverhalten zeichnen einen guten Wickel aus, damit die Kämmmaschine mit möglichst geringem Stillstand läuft. Diese Merkmale können nicht erreicht werden, wenn beim Wickelvorgang durch in den Bändern mitgeführte Luft Blasen auftreten. Hierdurch entstehen Verwerfungen zwischen den Wickellagen, wodurch der Wickel zum Teil unbrauchbar wird. Mit zunehmender Anzahl an Wattelagen steigt die Blasenbildung während des Wickelprozesses. Die Blasenbildung lässt sich durch eine geringere Wickelgeschwindigkeit vermeiden, was sich negativ auf die Produktivität auswirkt.

Es ist Aufgabe der Erfindung eine Wickelmaschine und ein Verfahren zum Wickeln von Watte zu schaffen, mit der ein qualitativ hochwertiger Wickel bei hoher Produktivität erzeugt wird.

Die Erfindung löst die gestellte Aufgabe durch die Lehre nach Anspruch 1 und 8; weitere vorteilhafte Ausgestaltungsmerkmale der Erfindung sind durch die Unteransprüche gekennzeichnet.

Gemäß der technischen Lehre nach Anspruch 1 umfasst die Wickelmaschine zum Wickeln von Watte mindestens eine Wickeleinheit, auf oder in der der Wickel gebildet wird. Es hat sich herausgestellt, dass die Blasenbildung und Verwerfungen durch eine Anpassung der Anspannung zwischen den Wickelwalzen beeinflussbar sind. Dies ist mit den Maschinen nach dem Stand der Technik aber nur schwer zu beeinflussen, da aufgrund des Antriebskonzeptes die Drehzahlverhältnisse zwischen den Wickelwalzen, Druckwalzen und Streckwerken starr sind.

Die Erfindung ist dadurch gekennzeichnet, dass jede Wickelwalze einen separaten Antriebsmotor aufweist, von denen mindestens einer Steuer- bzw. regelbar ist, und dass in Abhängigkeit des Wickeldurchmessers die Drehzahldifferenz zwischen den Wickelwalzen dynamisch einstellbar ist. Damit ist es möglich, bei der Erzeugung des Wickels eine Anspannung zwischen den Wickelwalzen zu erzeugen, die sich auf die Qualität des gebildeten Wickels auswirkt. Die durch die Blasenbildung hervorgerufenen Störungen wie Verwerfungen und Querfalten können reduziert und die Wickelmaschine mit einer höheren Geschwindigkeit betrieben werden.

Die Anspannungen können über Eingabeparameter an der Steuerung angepasst und während des Wickelvorganges überwacht und dynamisch geregelt werden.

Dabei werden die Wickelwalzen mit einer Drehzahldifferenz zwischen 0 bis 5 % betrieben, womit eine Wickelwalze mit einer bis zu 5 % geringeren Drehzahl betrieben wird. In Abhängigkeit des Wickeldurchmessers ( ≈ aufgewickelte Länge) kann die Anspannung dynamisch angepasst werden, so dass die Wickelqualität und Produktivität optimal eingestellt werden können.

Als besonders vorteilhaft hat sich eine Drehzahldifferenz von 1 bis 3 % herausgestellt, wobei die untere Wickelwalze um diese Differenz langsamer betrieben wird.

Um vollautomatisch während des laufenden Betriebes die Blasen, Querfalten und Verwerfungen zu vermeiden, wird durch einen Sensor der Ist-Durchmesser des Wattewickels ermittel. Durch einen Vergleich mit dem Soll-Durchmesser stellt die Steuerung fest, ob eine großflächige oder lokal kleinere Blasen durch mitgeschleppte Luft entstanden sind. Die Steuerung regelt die Antriebe der Wickelwalzen in der absoluten Drehzahl und/oder in der Drehzahldifferenz, bis die Abweichung des Durchmessers innerhalb eines Toleranzfeldes liegt, das beispielsweise bei 1 mm Durchmesserunterschied liegen kann. Der Soll-Durchmesser des Wattenwickels wird durch einen Sensor ermittelt, der die Anzahl der Umdrehungen des Wattenwickels an die Steuerung übermittelt. Durch die voreingestellte Faserqualität und die Dicke des Wattenbandes ermittelt die Steuerung den Soll-Durchmesser des Wattenwickels zu jedem Zeitpunkt. In Abhängigkeit der zu verarbeitenden Fasern verarbeitet die Steuerung auch Korrekturwerte für das Aufspringverhalten des Wattewickels, die Verformung des Wickels während des Aufwickelns und weitere materialabhängige Parameter.

In weiterer Ausführungsform weisen die Druckwalzen mindestens einen Antrieb auf, der unabhängig von den Wickelwalzen steuer- bzw. regelbar ist. Die Druckwalzen können dabei gemeinsam oder separat angetrieben werden. Damit ist es möglich, eine Anspannung zwischen den Druckwalzen und den Wickelwalzen zu erzeugen, mit dem ebenfalls die Qualität des Wattewickels beeinflussbar ist. Weiterhin können zur Wattentrennung beide Wickelwalzen den Wickel weiter antreiben, während die Watte zwischen mindestens einer stillstehenden Druckwalze abgerissen wird. Die Wickelmaschine arbeitet damit weitestgehend automatisch.

Das Verfahren zum Aufwickeln von Wattebändern zu Wattewickeln ist dadurch gekennzeichnet, dass beim Aufwickeln des Wattenbandes zwischen den Wickelwalzen eine Anspannung erzeugt wird, wobei in Abhängigkeit des Wickeldurchmessers die Drehzahldifferenz zwischen den Wickelwalzen dynamisch einstellbar ist. Die Anspannung reduziert insbesondere die Blasenbildung durch mitgeschleppte Luft, wobei diese durch eine Drehzahldifferenz von 0 bis -5 % zwischen den Wickelwalzen erzeugt wird, was einer Anspannung von 0 bis -5 % entspricht.

In bevorzugter Ausführungsform wird die Anspannung zwischen den Wickelwalzen durch eine Drehzahldifferenz von 1 bis 3 % erzeugt, was einer Anspannung von -1 bis -3 % entspricht.

Weitere vorteilhafte Ausführungsformen finden sich in den Unteransprüchen und werden durch die nachfolgende Beschreibung offenbart.

Unter Faserbänder werden im Sinne der Erfindung allgemein Watte, beispielsweise aus Baumwolle, Fasern, Vlies oder Flor bezeichnet, die in einer Spinnerei zum Zwischenlagern, Transport oder Weiterverarbeitung aufwickelbar sind. Die Faserbänder oder Streckenbänder werden im Tischkalander zu einem Watteband dubliert.

Die Erfindung wird nachfolgend anhand eines Ausführungsbeispieles näher erläutert. Es zeigt:
- Figur 1:: eine schematische Schnittdarstellung einer Wickelmaschine;
- Figur 2:: eine schematische Ansicht auf ein zugehöriges Streckwerk;
- Figur 3:: eine weitere schematische Darstellung einer Wickelmaschine.

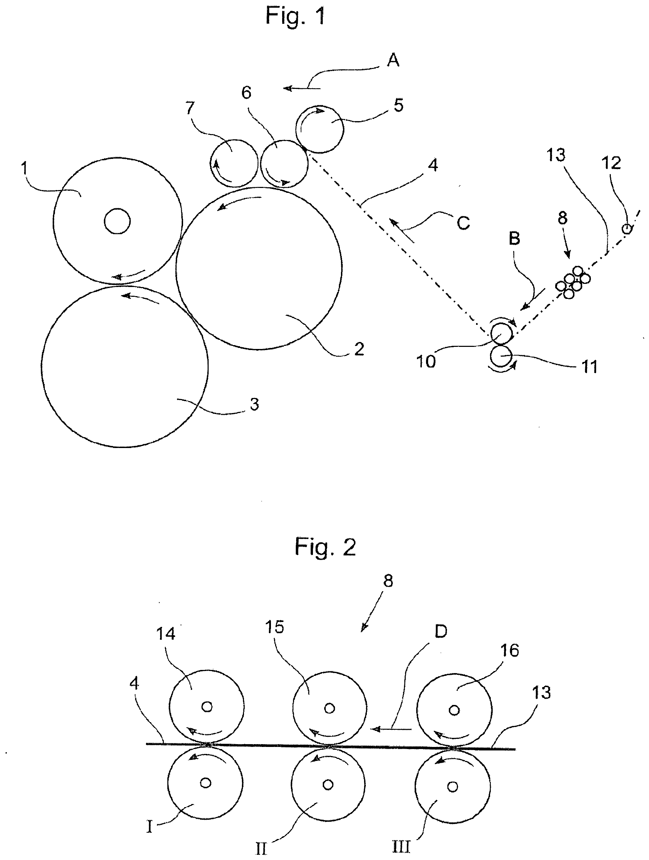

Nach Fig. 1 liegt ein Wattewickel 1 auf zwei in Pfeilrichtung drehbaren Wickelwalzen 2 und 3 (Wickelkalander) einer Bandwickelmaschine auf. Ein (strichpunktiert dargestelltes) Watteband 4 durchläuft einen aus drei Druckwalzen 5, 6 und 7 bestehenden Kalander (Druckkalander), bevor es dem Wattewickel 1 zuläuft und auf diesem aufgewickelt wird. Das Watteband 4 wird in einem Streckwerk 8 der Bandwickelmaschine aus einem oder mehreren zugeführten Faservliesen oder aus nebeneinanderliegenden Streckenbändern 13 gebildet. Dem Streckwerk 8 können noch ein Streckwerk 8' oder mehrere Streckwerke vorgeschaltet sein. Zwischen den Ausgangswalzen 14/I des Streckwerks 8 und der Druckwalze 5 des Druckkalanders ist ein aus zwei Walzen 10, 11 bestehender Tischkalander angeordnet. Den Eingangswalzen 16/III des Streckwerks 8 ist eine Umlenkrolle 12 vorgeordnet, die Faserbänder 13 aus vorgelagerten (nicht dargestellten) Faserbandkannen und einem (nicht dargestellten) Gatter umlenkt. Mit A ist die Arbeitsrichtung der Bandwickelmaschine bezeichnet. Die Pfeile B und C geben die Laufrichtung der verstreckten Streckenbänder 13 bzw. Wattenbänder 4 an. Die Drehrichtung der Walzen 2, 3, 5, 6, 7, 10 und 11 sowie des Wickels 1 ist durch gebogene Pfeile bezeichnet.

Nach Fig. 2 ist das Streckwerk 8 als 3-über-3-Streckwerk konzipiert, d. h. es besteht aus drei Unterwalzen I, II, III (I Ausgangs-Unterwalze, II Mittel-Unterwalze, III Eingangs-Unterwalze) und drei Oberwalzen 14, 15, 16. Im Streckwerk 8 erfolgt der Verzug des Faserverbandes aus mehreren Faserbändern 13. Der Verzug setzt sich zusammen aus Vorverzug und Hauptverzug. Die Walzenpaare 16/III und 15/II bilden das Vorverzugsfeld, und die Walzenpaare 15/II und 14/I bilden das Hauptverzugsfeld. Mit D ist die Laufrichtung der Streckenbänder 13 innerhalb des Streckwerks 8 bezeichnet. Die verstreckten Streckenbänder 13 werden mittels der Kalanderwalzen 10, 11 in Richtung B als Wattenband 4 gezogen und in Richtung C umgelenkt. Die Drehrichtung der Walzen I, II, III, 14, 15, 16 ist durch gebogene Pfeile angegeben

Die Wickelwalzen 2, 3 weisen jeweils einen separaten Antrieb auf, wovon mindestens ein Antrieb Steuer- bzw. regelbar ist, so dass die Wickelwalzen 2, 3 mit gleichen oder unterschiedlichen Drehzahlen betrieben werden können. Um die Blasenbildung beim entstehenden Wattewickel 1 zu vermeiden, werden die Wickelwalzen 2, 3 mit einer Anspannung von 0 bis -5% angetrieben. Das heißt, dass eine Wickelwalze 2, 3 - bevorzugt die Wickelwalze 3 - mit einer bis zu 5 % geringeren Drehzahl betrieben wird, als die Wickelwalze 2. Bevorzugt werden die Wickelwalzen 2, 3 mit einer Anspannung von -1 bis -3% angetrieben. Es hat sich herausgestellt, dass sich der Wattewickel 1 beim Aufwickeln merklich verformt und die Tendenz hat, den Zwickel zwischen den Wickelwalzen 2 und 3 auszufüllen. Damit weicht die Außenkontur des Wattewickels 1 von der kreisrunden Form ab, wodurch die innerhalb der gleichen Zeit zurückgelegte Bogenlänge des Wattewickels 1 von der Bogenlänge der Wickelwalze 3 abweicht. Das hat zur Folge, dass beim Anfahren keine Verwerfungen oder Querfalten entstehen, die beim Abwickeln des Wattenbandes 1 sichtbar sind. Weiterhin wird beim größer werdenden Wattewickel 1 die Blasenbildung so deutlich reduziert, dass die Wickelmaschine mit höheren Geschwindigkeiten betrieben werden kann.

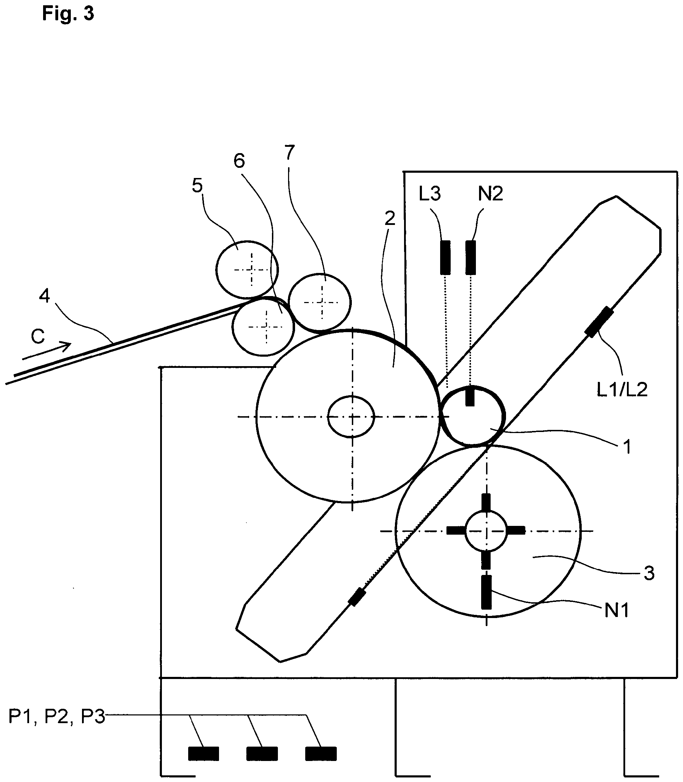

Nach Figur 3 sind in der Bandwickelmaschine verschiedene Sensoren angeordnet, mit denen die Qualität des Wickels im laufenden Betrieb überwacht werden kann.

Einer der wesentlichen Sensoren ist der Lasersensor L3, mit dem der Ist-Durchmesser des Wattewickels 1 während des Wickelvorganges gemessen werden kann. In der Darstellung ist nur ein Sensor L3 gezeigt. Um Abweichungen des Durchmessers über die Wickelbreite zu erfassen, können bevorzugt mehrere Sensoren L3 über die Breite angeordnet werden, oder ein Sensor L3 ist über die Breite des Wattewickels 1 verfahrbar angeordnet. Alternativ kann der Sensor L3 auch so ausgebildet sein, dass er die nahezu vollständige Breite des Wattewickels 1 erfassen kann. In der Steuerung der Maschine wird die Qualität der zu verarbeitenden Fasern eingegeben, so dass über die Einstellung der vorgelagerten Streckwerke 8, 8' und der Tischkalander 10, 11 die Dicke des Wattenbandes 4 feststeht. Über den Sensor N2, der als induktiver Näherungsschalter ausgebildet sein kann, wird die Anzahl der Umdrehungen des Wattewickels 1 ermittelt. Damit wird der momentane Soll-Durchmesser als Führungsgröße des Wattewickels 1 aus der Anzahl der Lagen und der Dicke des Wattenbandes 4 bestimmt und in der Steuerung mit dem Ist-Durchmesser verglichen. Ist der Ist-Durchmesser als Regelabweichung im Millimeterbereich größer als der Soll-Durchmesser, kann sich beim Wickeln eine Blase gebildet haben. Die Steuerung betätigt darauf hin als Stellglied einen Antrieb der Wickelwalzen 2, 3, wodurch beispielsweise die Anspannung zwischen den Wickelwalzen 2, 3 geändert werden kann, wodurch die Drehzahldifferenz zu oder abnehmen kann. Alternativ oder ergänzend können als Stellglieder beide Antriebe der Wickelwalzen 2, 3 gesteuert werden, so dass die Wickelgeschwindigkeit reduziert wird, bis der Soll-Durchmesser mit dem Ist-Durchmesser des Wattewickels 1 wieder in einem vorgegebenen Toleranzfeld liegt. Die entstehenden Blasen beim Wickeln können aufgrund von mitgeschleppter Luft lokal auftreten, wodurch eine oder mehrere voneinander getrennte Blasen zwischen der äußeren Lage des Wattenbandes 4 und dem Wattenwickel 1 entstehen. Wird dann mit gleicher Anspannung und Geschwindigkeit weitergefahren, kann daraus eine großflächige Blase entstehen, die das gebildete Wattenband 4 in unregelmäßige Streifen zerteilt, da die Luft entweichen möchte, wodurch aufgrund von Spannungen durch die gesammelte Luft Lücken, Verwerfungen, Fehlstellen und Unregelmäßigkeiten entstehen, die sich beim Kämmen negativ auswirken können. Durch die Luftblasen kann sich lokal der Durchmesser des Wattenwickels 1 um mehrere Millimeter vergrößern. Die mitgeschleppte Luft kann das Wattenband 4 bis zu 10 mm vom Wattewickel 1 abheben lassen, was über den oder die Lasersensoren L3 deutlich zu ermitteln ist. Die Steuerung der Bandwickelmaschine ist mit einem Toleranzfeld versehen, so dass Abweichungen beispielsweise im Bereich bis zu 1 Millimeter sich nicht auf die Anspannung und die Wickelgeschwindigkeit auswirken, da kleine Luftblasen sich auch wieder auflösen können.

Über die Sensoren L1 und L2, die auch als Lasersensoren ausgebildet sein können, kann der Zylinderhub der Wickelwalzen 2, 3 auf jeder Maschinenseite kontrolliert werden, um ein Verkippen bzw. eine einseitige Druckbelastung zu vermeiden. Damit wird ein einseitiges Aufwickeln vermieden.

Mit dem Sensor N1 wird die Anzahl der Umdrehungen der Wickelwalze 3 bestimmt, wobei der Sensor N1 als induktiver Näherungsschalter ausgebildet sein kann. Über diesen Sensor wird die Wickellänge bestimmt, so dass die Bandwickelmaschine kurz vor beispielsweise 300 m Wickellänge in der Geschwindigkeit runterfährt, um den fertigen Wattewickel auszustoßen und einen neuen Wattewickel zu starten.

Über die Sensoren P1, P2, P3, die als Drucksensoren ausgebildet sind, kann überprüft werden, ob der an der Bandwickelmaschine eingestellte Druck auch tatsächlich an den Wickelwalzen 2, 3 anliegt, so dass Störungen frühzeitig erkannt werden.

Über die separat zu steuernden bzw. regelnden Antriebe der Wickelwalzen 2, 3 kann der Abreissvorgang beim aufgerollten Wattewickel 1 modifiziert werden, indem durch ein etwas späteres Stoppen der Wickelwalzen 2 und 3 und ein gleichzeitiges Stoppen der Druckwalzen 5, 6, 7 das Wattenband 4 zwischen der Druckwalze 7 und der Wickelwalze 2 abgerissen wird, ein Ende aber noch zwischen der Wickelwalze 2 und der Druckwalze 7 verbleibt, wodurch die Bandwickelmaschine - nach dem eine neue Hülse eingelegt wurde - automatisch weiterfahren kann. Ein Einlegen des Wattenbandes 4 von Hand ist dadurch nicht mehr nötig.

Die zuvor genannten Verbesserungen sind auch mit den konventionellen Antrieben an den Druckwalzen 5, 6, 7 und Streckwerken 8, 8' möglich. Dabei werden die Druckwalzen 5, 6, 7 über einen gemeinsamen Antrieb mittels Zahnriemen oder Getrieberädern angetrieben, der unabhängig von den Antrieben der Wickelwalzen 2, 3 regelbar ist. Der Verzug innerhalb der Druckwalzen 5, 6, 7 ist damit voreingestellt und kann während des Betriebes nicht mehr eingestellt werden.

Auch die Walzen der Streckwerke 8, 8' sind in konventioneller Art miteinander durch ein Getriebe gekoppelt und werden durch einen separaten Antrieb angetrieben. Hier kann ebenfalls der voreingestellte Verzug nicht mehr im laufenden Betrieb eingestellt werden.

Als weitere Verbesserung können auch die Druckwalzen 5, 6, 7 mit je einem separaten und steuer- bzw. regelbaren Antrieb versehen sein, so dass bei Blasenbildung der Verzug zwischen den Druckwalzen 5, 6, 7, aber auch zwischen der Druckwalze 7 und der Wickelwalze 2 eingestellt werden kann. Der Prozess zur Erzeugung eines Wattewickels 1 kann damit weiter optimiert und stabilisiert werden, wodurch mit einer höheren Produktionsgeschwindigkeit gefahren werden kann.

Weiterhin kann damit auch der Verzug zwischen den Druckwalzen 5, 6 und dem vorgelagerten Streckwerk 8 beeinflusst werden, wodurch beispielsweise die Haarigkeit des Wattewickels beeinflusst werden kann.

### Bezugszeichen

- 1: Wattewickel
- 2: Wickelwalze
- 3: Wickelwalze
- 4: Wattenband
- 5: Druckwalze
- 6: Druckwalze
- 7: Druckwalze
- 8, 8': Streckwerk
- 10: Walze
- 11: Walze
- 12: Umlenkrolle
- 13: Streckenband
- 14/I: Ausgangswalzen
- 15/II: Walzen
- 16/III: Eingangswalzen

- A: Arbeitsrichtung der Wickelmaschine
- B: Laufrichtung Streckenband
- C: Laufrichtung Wattenband
- D: Laufrichtung Streckenband

- L1, 2, 3: Sensor
- N1, 2: Sensor
- P1, 2, 3: Sensor

## Patentansprüche

1. Wickelmaschine zum Aufwickeln von Wattebändern zu Wattewickeln, mit Wickelwalzen (2, 3) zum Tragen jeweils eines Wattewickels (1), mit den Wickelwalzen (2, 3) vorgeschalteten Druckwalzen (5, 6, 7) zum Kalandrieren eines zugeführten Wattenbandes (4), wobei den Druckwalzen (5, 6, 7) mindestens ein Streckwerk (8) mit Streckwerkswalzenpaaren vorgelagert ist, **dadurch gekennzeichnet, dass** jede Wickelwalze (2, 3) einen separaten Antrieb aufweist, wobei mindestens ein Antrieb über eine Steuerung steuer- bzw. regelbar ist, wobei mittels mindestens eines Sensors (L3) der Ist-Durchmesser des Wattewickels (1) während des Wickelvorganges ermittelt wird, und dass in Abhängigkeit des Ist-Wickeldurchmessers die Drehzahldifferenz zwischen den Wickelwalzen dynamisch einstellbar ist, indem die Antriebe der Wickelwalzen (2, 3) mit einer bis zu 5% unterschiedlichen Drehzahl betreibbar sind.

2. Wickelmaschine nach Anspruch 1 **dadurch gekennzeichnet, dass** die Antriebe der Wickelwalzen (2, 3) mit einer unterschiedlichen Drehzahl betreibbar sind, die zwischen 1 bis 3 % voneinander abweicht.

3. Wickelmaschine nach einem der vorhergehenden Ansprüche, **dadurch gekennzeichnet, dass** die Wickelmaschine einen weiteren Sensor (N2) aufweist, der die Anzahl der Umdrehungen des Wattewickels (1) ermittelt.

4. Wickelmaschine nach Anspruch 1, **dadurch gekennzeichnet, dass** die Druckwalzen (5, 6, 7) mindestens einen Antrieb aufweisen, der unabhängig von den Wickelwalzen (2, 3) steuer- bzw. regelbar ist.

5. Wickelmaschine nach Anspruch 1, **dadurch gekennzeichnet, dass** zum Abreißen des Wattenbandes (4) mindestens die Druckwalze (7) in der Drehzahl reduzierbar sind.

6. Wickelmaschine nach Anspruch 1, **dadurch gekennzeichnet, dass** die Wickelmaschine Sensoren (L1, L2) aufweist, mit denen der Zylinderhub der Wickelwalzen (2, 3) auf jeder Maschinenseite kontrollierbar ist.

7. Wickelmaschine nach Anspruch 1, **dadurch gekennzeichnet, dass** die Wickelmaschine Sensoren (P1-P3) aufweist, mit denen der an den Wickelwalzen (2, 3) anliegende Druck kontrollierbar ist.

8. Verfahren zum Aufwickeln von Wattebändern zu Wattewickeln, mit Wickelwalzen (2, 3), wobei der Wattewickel vor dem Aufwickeln verstreckt und kalandriert wird, **dadurch gekennzeichnet, dass** beim Aufwickeln des Wattenbandes (4) zwischen den Wickelwalzen (2, 3) eine Anspannung erzeugt wird, und dass der Ist-Durchmesser des Wattewickels (1) während des Wickelvorganges durch einen Sensor (L3) ermittelt und an eine Steuerung geleitet wird, und in Abhängigkeit des Ist-Wickeldurchmessers durch die Steuerung die Drehzahldifferenz zwischen den Wickelwalzen dynamisch einstellbar ist, in dem die Antriebe der Wickelwalzen (2, 3) mit einer bis zu 5% unterschiedlichen Drehzahl betreibbar sind.

9. Verfahren zum Aufwickeln von Wattebändern zu Wattewickeln nach Anspruch 8, **dadurch gekennzeichnet, dass** die Anspannung zwischen den Wickelwalzen (2, 3) durch eine Drehzahldifferenz von 1 bis 3 % erzeugt wird.

10. Verfahren zum Aufwickeln von Wattebändern zu Wattewickeln nach Anspruch 8, **dadurch gekennzeichnet, dass** die Anzahl der Umdrehungen des Wattewickels (1) durch einen weiteren Sensor (N2) ermittelt und an eine Steuerung geleitet wird.

11. Verfahren zum Aufwickeln von Wattebändern zu Wattewickeln nach einem der vorhergehenden Ansprüche 9 bis 10, **dadurch gekennzeichnet, dass** bei einer Abweichung des Ist-Durchmessers des Wattewickels von einem Soll-Durchmesser die Steuerung die Anspannung zwischen den Wickelwalzen (2, 3) verändert und/oder die Drehzahl der Wickelwalzen (2, 3) verändert.

12. Verfahren zum Aufwickeln von Wattebändern zu Wattewickeln nach Anspruch 11, **dadurch gekennzeichnet, dass** bei einer Abweichung des Ist-Durchmessers des Wattewickels von einem Soll-Durchmesser die Steuerung die Anspannung zwischen der Wickelwalze (2) und einer Druckwalze (7) verändert.

13. Verfahren zum Aufwickeln von Wattebändern zu Wattewickeln nach Anspruch 12, **dadurch gekennzeichnet, dass** zum Abreißen des Wattebandes mindestens die Druckwalze (7) in der Drehzahl reduziert wird.

## Claims

1. A winding machine for winding lap bands to form wound laps, with winding rolls (2, 3) for carrying respectively one wound lap (1), with pressure rolls (5, 6, 7) installed upstream the winding rolls (2, 3) for calendering a supplied lap band (4), wherein at least one drafting system (8) with a pair of drafting system rolls is installed upstream the pressure rolls (5, 6, 7), **characterized in that** each winding roll (2, 3) includes a separate drive, wherein at least one drive is open-loop controllable, respectively closed-loop controllable via a control, wherein at least one sensor (L3) determines the actual diameter of the wound lap (1) during the winding procedure, and **in that**, depending on the actual diameter of the wound lap, the rotational speed difference between the winding rolls is dynamically adjustable, **in that** the drives of the winding rolls (2, 3) are operable at a rotational speed having a difference of up to 5 %.

2. The winding machine according to claim 1, **characterized in that** the drives of the winding rolls (2, 3) are operable at a different rotational speed, which differs between 1 to 3 % from one another.

3. The winding machine according to any of the preceding claims, **characterized in that** the winding machine includes another sensor (N2), which determines the number of revolutions of the wound lap (1).

4. The winding machine according to claim 1, **characterized in that** the pressure rolls (5, 6, 7) have at least one drive, which is open-loop controllable, respectively closed-loop controllable independently of the winding rolls (2, 3).

5. The winding machine according to claim 1, **characterized in that** for tearing off the lap web (4), the rotational speed of at least the pressure roll (7) is reducible.

6. The winding machine according to claim 1, **characterized in that** the winding machine includes sensors (L1, L2), by means of which the cylinder stroke of the winding rolls (2, 3) is checkable on each side of the machine.

7. The winding machine according to claim 1, **characterized in that** the winding machine includes sensors (P1 to P3), by means of which the pressure applied to the winding rolls (2, 3) is checkable.

8. A method for winding lap bands to form wound laps, with winding rolls (2, 3), wherein the lap band is drafted and calendered prior to winding, **characterized in that** tension is generated between the winding rolls (2, 3) when winding the lap band (4), and **in that** the actual diameter of the wound lap (1) is determined during the winding procedure by means of a sensor (L3) and forwarded to a control, and **in that**, depending on the actual diameter of the wound lap, the rotational speed difference between the winding rolls is dynamically adjustable by means of the control, **in that** the drives of the winding rolls (2, 3) are operable at a rotational speed having a difference of up to 5 %

9. The method for winding lap bands to form wound laps according to claim 8, **characterized in that** the tension between the winding rolls (2, 3) is generated by a difference in the rotational speed of 1 to 3 %.

10. The method for winding lap bands to form wound laps according to claim 8, **characterized in that** the number of revolutions of the wound lap (1) is determined by a further sensor (N2) and forwarded to a control.

11. The method for winding lap bands to form wound laps according to any of the preceding claims 9 to 10, **characterized in that**, in the event of the actual diameter of the wound lap deviating from a nominal diameter, the control modifies the tension between the winding rolls (2, 3) and/or modifies the rotational speed of the winding rolls (2, 3).

12. The method for winding lap bands to form wound laps according to claim 11, **characterized in that**, in the event the actual diameter of the wound lap deviates from a nominal diameter, the control modifies the tension between the winding roll (2) and a pressure roll (7).

13. The method for winding lap webs to form wound laps according to claim 12, **characterized in that**, for tearing off the lap band, the rotational speed of at least the pressure roll (7) is reduced.

## Revendications

1. Machine enrouleuse pour enrouler des rubans de nappe pour former des rouleaux de nappe, avec des rouleaux-enrouleurs (2, 3) pour porter respectivement un rouleau de nappe (1), avec des rouleaux de pression (5, 6, 7) montés en amont des rouleaux-enrouleurs (2, 3) pour calandrer un ruban de nappe (4) amené, dans laquelle au moins un train d'étirage (8) avec des paires de rouleaux de train d'étirage est monté en amont des rouleaux de pression (5, 6, 7), **caractérisée en ce que** chaque rouleau-enrouleur (2, 3) comprend un entraînement séparé, dans laquelle au moins un entraînement est contrôlable, respectivement réglable par l'intermédiaire d'une commande, dans laquelle le diamètre actuel du rouleau de nappe (1) est détecte pendant le procédé d'enroulement par l'intermédiaire d'au moins un capteur (L3), et **en ce que** la différence de la vitesse de rotation entre les rouleaux-enrouleurs est dynamiquement ajustable en fonction du diamètre actuel de rouleau de nappe en étant capable d'opérer les entraînements des rouleaux-enrouleurs (2, 3) à une vitesse de rotation différente jusqu'à concurrence de 5 %.

2. Machine enrouleuse selon la revendication 1, **caractérisée en ce que** les entraînements des rouleaux-enrouleurs (2, 3) sont opérables à une vitesse de rotation, qui s'écarte entre 1 à 3 % l'une de l'autre.

3. Machine enrouleuse selon l'une des revendications précédentes, **caractérisée en ce que** la machine enrouleuse comprend un autre capteur (N2), lequel détecte le nombre de tours du rouleau de nappe (1).

4. Machine enrouleuse selon la revendication 1, **caractérisée en ce que** les rouleaux de pression (5, 6, 7) comprennent au moins un entraînement, lequel est contrôlable, respectivement réglable indépendamment des rouleaux-enrouleurs (2, 3).

5. Machine enrouleuse selon la revendication 1, **caractérisée en ce que** la vitesse de rotation au moins du rouleau de pression (7) est réductible pour rompre le ruban de nappe (4).

6. Machine enrouleuse selon la revendication 1, **caractérisée en ce que** la machine enrouleuse comprend des capteurs (L1, L2) avec lesquels la course de cylindre des rouleaux-enrouleurs (2, 3) est maitrisable de chaque côté de machine.

7. Machine enrouleuse selon la revendication 1, **caractérisée en ce que** la machine enrouleuse comprend des capteurs (P1 à P3) avec lesquels la pression appliquée aux rouleaux-enrouleurs (2, 3) est maitrisable.

8. Méthode pour enrouler des rubans de nappe pour former des rouleaux de nappe, avec des rouleaux-enrouleurs (2, 3), dans laquelle le ruban de nappe est étiré et calandré avant l'enroulement, **caractérisée en ce que**, pendant l'enroulement du ruban de nappe (4), une tension est générée entre les rouleaux-enrouleurs (2, 3) et **en ce que** le diamètre actuel du rouleau de nappe (1) est déterminé pendant le procédé d'enroulement par l'intermédiaire d'un capteur (L3) et acheminé à une commande, et la différence de vitesse de rotation entre les rouleaux-enrouleurs est dynamiquement ajustable par la commande en fonction du diamètre actuel de rouleau de nappe en étant capable d'opérer les entraînements des rouleaux-enrouleurs (2, 3) à une vitesse de rotation différente jusqu'à concurrence de 5 %.

9. Méthode pour enrouler des rubans de nappe pour former des rouleaux de nappe selon la revendication 8, **caractérisée en ce que** la tension entre les rouleaux-enrouleurs (2, 3) est générée par une différence de vitesse de rotation de 1 à 3 %.

10. Méthode pour enrouler des rubans de nappe pour former des rouleaux de nappe selon la revendication 8, **caractérisée en ce que** le nombre de tours du rouleau de nappe (1) est détecté par un autre capteur (N2) et acheminé à une commande.

11. Méthode pour enrouler des rubans de nappe pour former des rouleaux de nappe selon l'une des revendications précédentes 9 à 10, **caractérisée en ce que**, en cas d'un écartement du diamètre actuel du rouleau de nappe d'un diamètre nominal, la commande modifie la tension entre les rouleaux-enrouleurs (2, 3) et/ou modifie la vitesse de rotation des rouleaux-enrouleurs (2, 3).

12. Méthode pour enrouler des rubans de nappe pour former des rouleaux de nappe selon la revendication 11, **caractérisée en ce que**, en cas d'un écartement du diamètre actuel du rouleau de nappe d'un diamètre nominal, la commande modifie la tension entre le rouleau-enrouleur (2) et un rouleau de pression (7).

13. Méthode pour enrouler des rubans de nappe pour former des rouleaux de nappe selon la revendication 12, **caractérisée en ce que** la vitesse de rotation d'au moins du rouleau de pression (7) est réduite pour rompre le ruban de nappe.
